# EUROPÄISCHE PATENTSCHRIFT

(11) **EP 0 333 649 B1**
(45) Veröffentlichungstag und Bekanntmachung des Hinweises auf die Patenterteilung: **08.06.1994**
(21) Anmeldenummer: 89810134.0
(22) Anmeldetag: 21.02.1989
(51) Int. Cl.: C07D 493/10, G03C 1/73, B41M 5/124, B41M 5/26

(54) **3-Basisch substituierte 2-Methylfluorane**
3-Basically substituted 2-methyl fluoranes
Méthyl-2 fluoranne substitué par un groupe basique en position 3

(30) Priorität: 01.03.1988 CH 766/88
(43) Veröffentlichungstag der Anmeldung: 20.09.1989
(73) Patentinhaber: CIBA-GEIGY AG, 4002 Basel (CH)
(72) Erfinder: Zink, Rudolf, CH-4106 Therwil (CH)

(56) Entgegenhaltungen:
- DE-A- 3 531 272
- Chemical Abstracts, Band 100, Nr. 12, 19. März 1984, Columbus, Ohio, USA; Ricoh Co. Ltd.: "Fluoran color former", Seite 79, Spalte 2, Zusammenfassung Nr. 87259w

## Beschreibung

Die vorliegende Erfindung betrifft 3-basisch substituierte 2-Methylfluorane, Verfahren zu ihrer Herstellung und ihre Verwendung als Farbbildner in druckempfindlichen oder wärmeempfindlichen Aufzeichnungsmaterialien.

Verbindungen aus der Klasse der 3,7-Diaminofluorane, die als Farbbildner in Aufzeichnungmaterialien Verwendung finden, sind aus Chemical Abstacts, Band 100, Nr. 12, nos 87257u, 87258v und 87259w bekannt.

Die erfindungsgemässen 3-basisch substituierten 2-Methylfluorane entsprechen der allgemeinen Formel 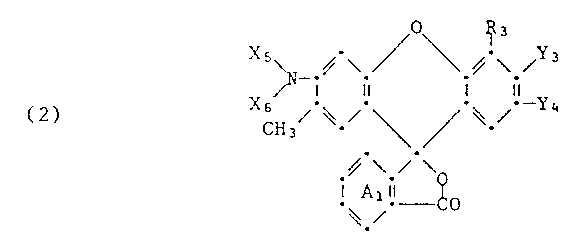
worin
R₃ Wasserstoff, Halogen oder C₁-C₅-Alkyl,
Y₃ Wasserstoff, Halogen, C₁-C₅-Alkyl, C₁-C₅-Alkoxy oder 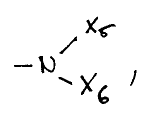
Y₄ Halogen, C₁-C₅-Alkyl oder 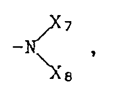
X₅, X₆, X₇ und X₈, unabhängig voneinander, je C₁-C₈-Alkyl, C₅-C₆-Cycloalkyl, Tetrahydrofurfuryl oder unsubstituiertes oder durch Halogen, Cyano, C₁-C₅-Alkyl, Trifluormethyl, C₁-C₅-Alkoxy oder C₁-C₅-Alkoxycarbonyl substituiertes Benzyl oder Phenyl und X₈ auch Wasserstoff oder die Substituentenpaare (X₅ und X₆) und (X₇ und X₈), unabhängig voneinander, je zusammen mit dem jeweiligen gemeinsamen Stickstoffatom Pyrrolidino, Piperidino oder Morpholino bedeuten und der Ring A₁ unsubstituiert oder durch Halogen, C₁-C₅-Alkoxycarbonyl oder Di-C₁-C₅-Alkylamino substituiert ist.

Alkyl, Alkoxy und Alkylthio stellen bei der Definition der Reste der Fluoranverbindungen solche Gruppen oder Gruppenbestandteile dar, die 1 bis 5, insbesondere 1 bis 3 Kohlenstoffatome aufweisen. Beispiele für derartige Gruppen sind Methyl, Ethyl, n-Propyl, Isopropyl, n-Butyl, sek.-Butyl, tert.Butyl, Amyl, Isoamyl oder tert.Amyl bzw. Methoxy, Ethoxy, Isopropoxy, Isobutoxy oder tert.Butoxy bzw. Methylthio, Ethylthio, Propylthio oder Butylthio.

Halogen bedeutet beispielsweise Fluor, Brom oder vorzugsweise Chlor.

Stellen die Substitutenten X⁵, X⁶, X⁷ und X⁸ Alkylgruppen dar, so können sie geradkettig oder verzweigt sein. Beispiele solcher Alkylreste sind Methyl, Ethyl, n-Propyl, Isopropyl, n-Butyl, sek.-Butyl, tert.-Butyl, 1,1,3,3-Tetramethylbutyl, Amyl, Isoamyl, n-Hexyl, 2-Ethyl-hexyl, n-Heptyl, n-Octyl, oder Isooctyl.

Beispiele für Cycloalkyl in der Bedeutung der X-Reste sind Cyclopentyl, Cycloheptyl oder Vorzugsweise Cyclohexyl. Die Cycloalkylreste können einen oder mehrere C₁-C₄-Alkylreste, vorzugsweise Methylgruppen, enthalten und weisen insgesamt 5 bis 10 Kohlenstoffatome auf.

Unter den Fluoranverbindungen der Formel (2) sind diejenigen, in denen Y₃ Wasserstoff, Halogen oder Methyl und Y₄ 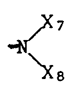
bedeuten, bevorzugt.

X₅ und X₆ sind vorzugsweise C₁-C₆-Alkyl, Cyclohexyl, Benzyl oder Tolyl, oder -NX₅X₆ ist bevorzugt auch Pyrrolidinyl oder N-C₁-C₅-Alkyl-N-tetrahydrofurfurylamino.

Bei Y₄ ist X₇ vorzugsweise C₁-C₈-Alkyl, Benzyl, Phenyl oder durch Halogen, Methyl, Methoxy oder Trifluormethyl substituiertes Phenyl oder Benzyl und X₈ Wasserstoff, C₁-C₅-Alkyl, Benzyl oder durch Halogen, Methyl, Methoxy oder Trifluormethyl substituiertes Benzyl.

Von besonderem Interesse sind folgende drei Gruppen von Fluoranverbindungen.

### 1. Fluoranverbindungen der Formel

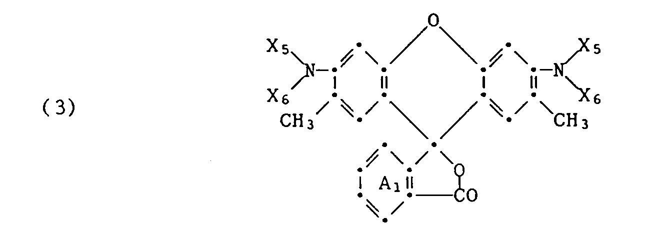

### 2. Fluoranverbindungen der Formel

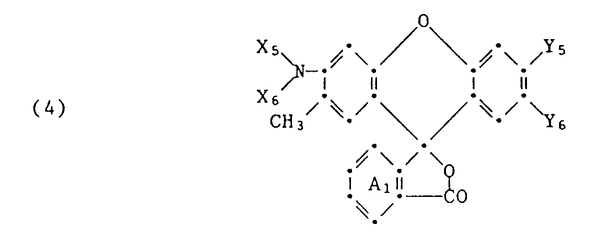

### 3. Fluoranverbindungen der Formel

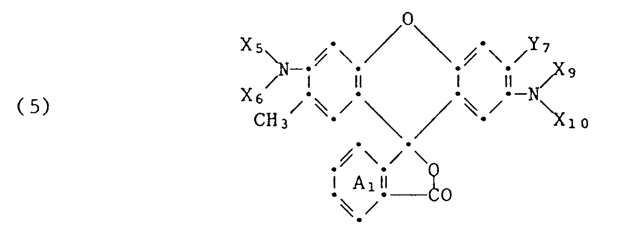

In den Formeln (3), (4) und (5) haben A₁, X₅ und X₆ die oben angegebenen Bedeutungen, Y₅ ist Wasserstoff, C₁-C₅-Alkyl oder Halogen, Y₆ ist Halogen oder C₁-C₅-Alkyl, Y₇ ist Wasserstoff, Halogen, C₁-C₅-Alkyl oder C₁-C₅-Alkoxy, X₉ ist C₁-C₈-Alkyl, Benzyl, Phenyl oder durch Halogen, Methyl, Methoxy, Carbomethoxy oder Trifluormethyl substituiertes Phenyl oder Benzyl und X₁₀ ist Wasserstoff, C₁-C₅-Alkyl, Benzyl oder durch Halogen, Methyl, Methoxy₇ Carbomethoxy oder Trifluormethyl substituiertes Benzyl.

Besonders bevorzugt sind Fluoranverbindungen der Formel (5), in der Y₇ Wasserstoff oder Methyl, X₉ Phenyl oder durch Halogen, Methyl oder Trifluormethyl substituiertes Phenyl und X₁₀ Wasserstoff bedeuten.

Ganz besonders bevorzugt sind Fluoranverbindungen der Formel (3), in der X₅ und X₆ C₁-C₅-Alkyl sind und der Ring A₁ durch Halogen (1 bis 4 Chloratome) substituiert ist.

In den Formeln (2) bis (5) bedeuten X₅ und X₆ insbesondere je Methyl.

Die erfindungsgemässen Fluoranverbindungen der Formeln (2) bis (5) stellen neue chromogene Verbindungen dar und können nach an sich bekannten Methoden hergestellt werden.

Ein Verfahren zur Herstellung der Fluoranverbindungen der Formel (2) besteht darin, dass man ein Mol eines Anhydrids der Formel 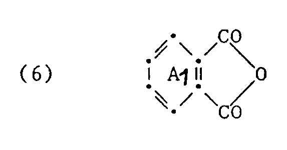
mit einem Mol einer Verbindung der Formel 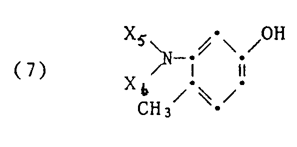
und einem Mol einer Verbindung der Formel 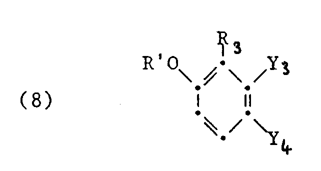
umsetzt, worin in Formeln (6), (7) und (8) A₁, X⁵, X⁶, Y³, Y⁴ und R₃ die angegebene Bedeutung haben und R' Wasserstoff oder Methyl bedeutet.

Vorteilhafterweise werden die erfindungsgemässen Laktone dadurch hergestellt, dass man eine Ketosäureverbindung der Formel 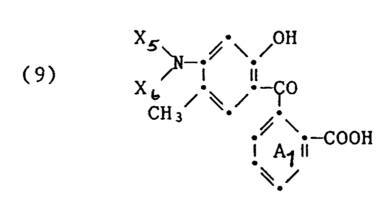
mit einer Verbindung der Formel (8) umsetzt, worin X⁵, X⁶ und A₁ die angegebene Bedeutung haben.

Die Umsetzungen werden vorzugsweise so ausgeführt, dass man die Reaktionskomponenten in Anwesenheit eines sauren Kondensationsmittels bei einer Temperatur von 20 bis 140°C zur Reaktion bringt. Beispiele für derartige Kondensationsmittel sind Essigsäureanhydrid, Zinkchlorid, Aluminiumchlorid, Schwefelsäure, Phosphorsäure und Phosphoroxychlorid. Setzt man das Phthalsäureanhydrid der Formel (6) mit Phenolverbindungen der Formeln (7) und (8) um, die identisch sind, so können Fluoranverbindungen vom Typ der Formel (3) direkt hergestellt werden.

Die Isolierung des Endproduktes der Formel (2) erfolgt in allgemein bekannter Weise durch Einstellen des Reaktionsgemisches auf einen pH-Wert von mindestens 6, vorzugsweise 7 bis 14, z.B. mit Alkalien, wie z.B. Alkalimetallhydroxiden, Ammoniak, Alkalimetallcarbonaten oder -bicarbonaten und Abtrennen des gebildeten Produktes, Waschen und Trocknen oder durch Behandeln mit geeigneten organischen Lösungsmitteln, wie z.B. Methanol, Isopropanol, Benzol, Chlorbenzol, Toluol oder Xylol. Nötigenfalls kann bei dem Umkristallisieren der Fluoranverbindungen auch Petrolether, Wasser oder Ammoniakwasser mitverwendet werden.

Die Ausgangsprodukte der Formeln (6), (7) und (8) sind grösstenteils bekannt.

Die Zwischenprodukte der Formel (9) sind neu. Sie werden durch Umsetzung des Säureanhydrids der Formel (6) mit einer Verbindung der Formel (7) vorzugsweise in Gegenwart eines der genannten Kondensationsmittel und gegebenenfalls in einem organischen Lösungsmittel z.B. Benzol, Toluol, Xylol oder Chlorbenzol erhalten.

Die Fluoranverbindungen der Formeln (2) bis (5) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach Bedeutung von Y₃ und Y₄ und dem verwendeten Entwickler intensive gelbe, orange, rote, violette, grüne, blaue, grün-blaue, graugrüne, graue oder schwarze Farbtöne, die besonders licht- und sublimationsecht sind.

Die Fluoranverbindungen der Formeln (2) bis (5) sind auch sehr wertvoll im Gemisch mit einem oder mehreren anderen bekannten Farbbildnern, z.B. 3,3-(Bis-aminophenyl-)-phthaliden, 3-Indolyl-3-aminophenylazaphthaliden, (3,3-Bis-indolyl-)-phthaliden, 3-Amino-fluoranen, 3-Dialkylamino-7-dibenzylaminofluoranen, 3-Dialkylamino-6-methyl-7-arylaminofluoranen, 3,6-Bisalkoxyfluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um graue oder schwarze Färbungen zu ergeben.

Die Fluoranverbindungen der Formeln (2) bis (5) zeigen sowohl auf aktivierten Tonen, wie auch auf phenolischen Unterlagen eine ausgezeichnete Farbintensität. Sie eignen sich insbesondere als sich schnell entwickelnde Farbbildner für die Verwendung in einem wärmeempfindlichen oder insbesondere druckempfindlichen Aufzeichnungsmaterial, das sowohl Kopierals auch Registriermaterial sein kann. Sie zeichnen sich dadurch aus, dass sie pH stabil, lichtecht und in den Kapselölen gut löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formeln (2) bis (5) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, Zirkondioxyd, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-(α,α-dimethylbenzyl-)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfstoffen wie Kieselgel oder UV-Absorbern, wie z.B. 2-(2′-Hydroxyphenyl-)benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-C₁-C₃-alkylierte Diphenylalkane, Dodecylbenzol, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (2) bis (5) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (2) bis (5) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der

Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12,51,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 2,4-Dihydroxydiphenylsulfon, 4'-Hydroxy-4-methyldiphenylsulfon, 4'-Hydroxy-4-isopropoxydiphenylsulfon, 4-Hydroxy-acetophenon, 2,2'-Dihydroxydiphenyl, 4,4'-Cyclohexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyonet, 4,4-Bis-(4-hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Fluoranverbindungen und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, carboxylierte Butadien-Styrolcopolymerisate, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Bis-Stearoyl-ethylendiamid, Stearinsäureamid, Phthalsäureanhydrid, Metallstearate, wie z.B. Zinkstearat, Phthalsäurenitril, Dimethylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formeln (2) bis (5) ist die Herstellung eines Farbbildes mittels photohärtbarer Mikrokapseln, wie sie z.B. in der DE-OS 3 247 488 beschrieben sind. In den folgenden Beispielen beziehen sich die angegebenen Prozentsätze, wenn nichts anderes angegeben ist, auf das Gewicht. Teile bedeuten Gewichtsteile.

### Beispiel 1:

(a) 30,2 g 3-Dimethylamino-4-methylphenol werden in 50 ml Toluol bei 20°C gelöst. Unter Rühren gibt man 37 g Phthalsäureanhydrid und 0,2 g Zinkchlorid zu und heizt auf 100-105°C. Diese Temperatur wird während 9 Stunden gehalten. Alsdann versetzt man das Reaktionsprodukt mit 2N Natriumhydroxidlösung, worauf zwei Phasen gebildet werden. Die alkalische Wasser-Phase wird abgetrennt und mit Salisäure neutralisiert. Man erhält einen Niederschlag, der nach Lösen in 500 ml 1N Natriumhydroxidlösung und Zugabe von Natriumchlorid nochmals isoliert wird. Die Umkristallisation aus wässeriger Essigsäure ergibt das 2-Hydroxy-4-dimethylamino-5-methyl-2'-carboxy-benzophenon mit Smp. 186-188°C (Zersetzung).
(b) 7,5 g der gemäss a) isolierten Ketosäure werden in 25 ml Schwefelsäure (98 %) bei 25°C gelöst. In diese Lösung werden bei 15-20°C 5,3 g 4-Methoxy-2-methyl-diphenylamin im Verlauf einer Stunde eingetragen, worauf das Gemisch 4 Stunden bei 15-20°C gerührt wird. Man giesst das Reaktionsprodukt auf eine Mischung aus 30 ml Wasser und 50 ml Xylol und hält den pH-Wert zwischen 12 und 14 durch gleichzeitiges Zudosieren von 150 ml 40%iger Natriumhydroxidlösung. Man rührt die Emulsion noch während 1/2 Stunde bei 90-95°C und trennt die wässrige Phase ab. Die Xylolphase wird mit Aktivkohle klärfiltriert und eingeengt, wobei 6,3 g der Verbindung der Formel 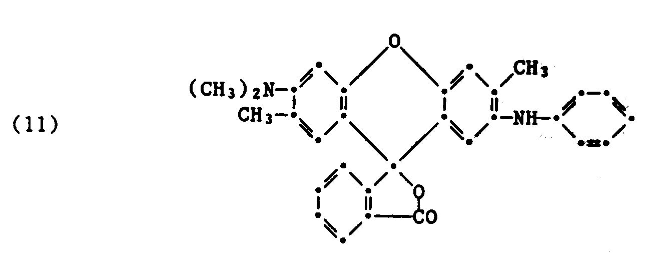 erhalten werden. Nach Umkristallisation aus Toluol/Petrolether zeigt die Verbindung einen Schmelzpunkt von 186-187°C.
   Auf säuremodifiziertem Ton erzeugt diese Fluorenverbindung eine rötlichschwarze, auf Phenolharz eine grünlich-schwarze Farbe.

### Beispiel 2:

Verwendet man in Beispiel 1(b) anstelle von 4-Methoxy-2-methyl-diphenylamin 4,7 g 4-tert.Butylphenol und verfährt man wie im Beispiel 1 beschrieben, so erhält man 1,7 g einer Fluoranverbindung der Formel 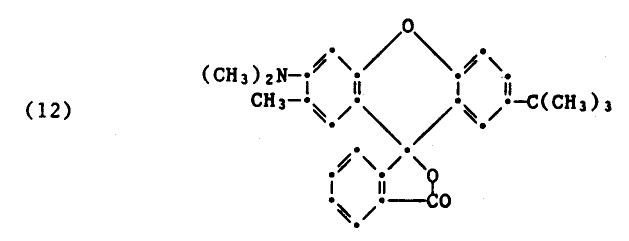
welche nach Umkristallisieren aus Methanol/Ammoniakwasser einen Schmelzpunkt von 170-171°C aufweist.

Auf säuremodifiziertem Ton erzeugt diese Fluoranverbindung eine lichtechte, rotorange Farbe.

### Beispeil 3:

Die gemäss Beispiel 1(a) erhaltene Toluolphase wird zur Trockne eingeengt. Man erhält 5,6 g einer Verbindung der Formel 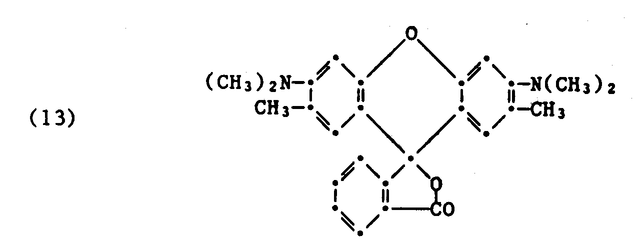
Nach Umkristallisation aus Aceton/Ammoniakwasser und anschliessend aus Toluol ist der Schmelzpunkt dieser Fluoranverbindung 224-225°C. Auf säuremodifiziertem Ton und auf Phenolharz erzeugt diese Fluoranverbindung sofort eine intensiv violette Farbe.

### Beispeil 4:

(a) 30,2 g 3-Dimethylamino-4-methyl-phenol werden in 50 ml Toluol bei 20°C gelöst. Unter Rühren gibt man in die Lösung 28,6 g Tetrachlorphthalsäureanhydrid und 0,5 g Zinkchlorid zu. Man heizt auf Rückfluss und lässt während 20 Stunden bei Rückflusstemperatur reagieren. Hierauf wird das Reaktionsprodukt auf 20°C abgekühlt und der erhaltene Niederschlag abfiltriert. Nach Trocknen und Umkristallisieren aus 60%iger Essigsäure erhält man 32 g der Ketosäure der Formel 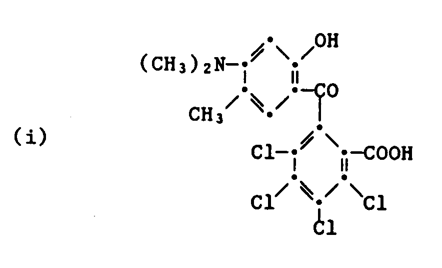 mit einem Schmelzpunkt von 197-198°C.
(b) Das gemäss (a) erhaltene Filtrat wird eingeengt. Der Rückstand wird in Aceton gelöst, klärfiltriert, und dann das Aceton abdestilliert. Man erhält 7,5 g Rohprodukt, welches durch Chromatographie an Silicagel mit 9 Teilen Toluol und 1 Teil Essigsäureethylester gereinigt wird. Man erhält eine Fluoranverbindung der Formel 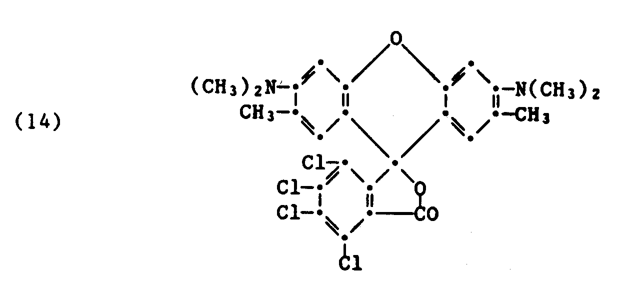 welche nach Umkristallisation aus Isopropylalkohol/Wasser farblose Kristalle mit einem Schmelzpunkt von 285°C (unter Zersetzung) ergibt. Auf säuremodifiziertem Ton erzeugt diese Fluoranverbindung sofort eine blaue Farbe.

### Beispiel 5:

(a) Verwendet man in Beispiel 4 21,7 g 4,5-Dichlorphthalsäureanhydrid anstelle von Tetrachlorphthalsäureanhydrid und verfährt ansonst wie in Beispiel 4 angegeben, erhält man 7,7 g der Ketosäure der Formel 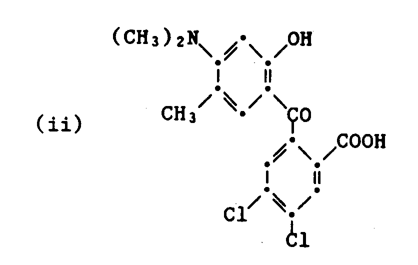 Smp. 196-200°C.
(b) Analog Beispiel 4(b) erhält man aus dem eingeengten Filtrat und nach Umkristallisation des Rückstandes aus Isopropanol und Toluol (4:1) die Fluoranverbindung der Formel 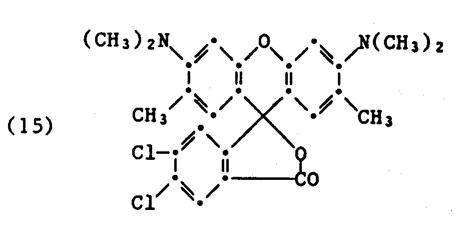 mit einem Schemlzpunkt von 283-285°C. Auf säuremodifizierten Ton ergibt dieser Farbbildner eine aussserordentlich farbstarke, rotviolette Färbung.

### Beispeil 6:

### Herstellung eines druckempfindlichen Kopierpapiers

Eine Lösung von 1 g der Fluoranverbindung der Formel (14) (Beispiel 4) in 80 g Diisopropylnaphthalin und 19 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensiv blaue Kopie, die ausgezeichnet lichtecht ist.

### Beispiel 7:

1 g der Fluoranverbindung der Formel (12) gemäss Beispiel 2 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 »m Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensiv rotorange Farbe.

### Beispiel 8:

### Herstellung eines wärmeempfindlichen Aufzeichnungsmaterials

In einer Kugelmühle werden 32 g 4,4′Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 »m beträgt. In einer zweiten Kugelmühle werden 6 g der Fluoranverbindung der Formel (11) gemäss Beispiel 1, 3 g eines zu 88 % hydrolysierten Polyvinylalkohols und 60 ml Wasser zu einer Teilchengrösse von ca. 3 »m gemahlen.

Die beiden Dispersionen werden zusammengegeben und mit einem Trockenauftragsgewicht von 5,5 g/m² auf ein Papier gestrichen. Durch Berührung des Papiers mit einem erhitzten Metallstift wird eine intensive und lichtechte schwarze Farbe erhalten.

### Beispiel 9:

In einer Kugelmühle werden 32 g 4,4′Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 »m beträgt. In einer zweiten Kugelmühle werden 3 g der Fluoranverbindung der Formel (11) gemäss Beispiel 1 zusammen mit 3 g 3-Diethylamino-6-methyl-7-anilinofluoran, 3 g eines zu 88 % hydrolysierten Polyvinylalkohols und 60 ml Wasser zu einer Teilchengrösse von ca. 3 »m gemahlen.

Die beiden Dispersionen werden zusammengegeben und mit einem Trockenauftragsgewicht von 5,5 g/m² auf ein Papier gestrichen, welches trotz hoher Reaktivität praktisch keine Hintergrundverfärbung aufweist. Durch Berührung des Papiers mit einem erhitzten Metallstift wird eine intensive und lichtechte schwarze Farbe erhalten.

## Patentansprüche

1. 2-Methylfluoranverbindung, dadurch gekennzeichnet, dass sie der Formel 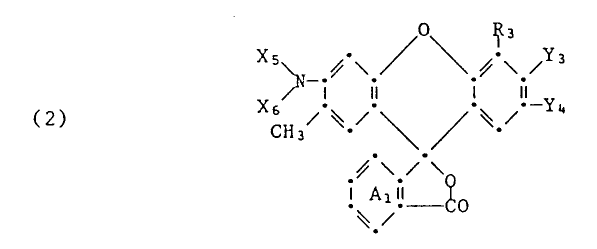 entspricht, worin
R₃ Wasserstoff, Halogen oder C₁-C₅-Alkyl,
Y₃ Wasserstoff, Halogen, C₁-C₅-Alkyl, C₁-C₅-Alkoxy oder 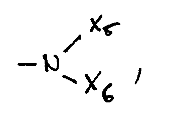 Y₄ Halogen, C₁-C₅-Alkyl oder 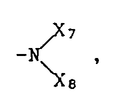 X₅, X₆, X₇ und X₈, unabhängig voneinander, je C₁-C₈-Alkyl, C₅-C₆-Cycloalkyl, Tetrahydrofurfuryl oder unsubstituiertes oder durch Halogen, Cyano, C₁-C₅-Alkyl, Trifluormethyl, C₁-C₅-Alkoxy oder C₁-C₅-Alkoxycarbonyl substituiertes Benzyl oder Phenyl und X₈ auch Wasserstoff oder die Substituentenpaare (X₅ und X₆) und (X₇ und X₈), unabhängig voneinander, je zusammen mit dem jeweiligen gemeinsamen Stickstoffatom Pyrrolidino, Piperidino oder Morpholino bedeuten und der Ring A₁ unsubstituiert oder durch Halogen, C₁-C₅-Alkoxycarbonyl oder Di-C₁-C₅-Alkyl amino substituiert ist.

2. 2-Methylfluoranverbindung gemäss Anspruch 1, dadurch gekennzeichnet, dass in Formel (2) Y₃ Wasserstoff, Halogen oder Methyl und Y₄ -NX₇X₈ bedeuten.

3. 2-Methylfluoranverbindung gemäss einem der Ansprüche 1 oder 2, dadurch gekennzeichnet, dass in Formel (2) X₇ C₁-C₈-Alkyl, Benzyl, Phenyl oder durch Halogen, Methyl, Methoxy oder Trifluormethyl substituiertes Phenyl oder Benzyl und X₈ Wasserstoff, C₁-C₅-Alkyl, Benzyl oder durch Halogen, Methyl, Methoxy oder Trifluormethyl substituiertes Benzyl bedeuten.

4. 2-Methylfluoranverbindung gemäss einem der Ansprüche 1 bis 3, dadurch gekennzeichnet, dass in Formel (2) X₅ und X₆, unabhängig voneinander, je C₁-C₆-Alkyl, Cyclohexyl, Benzyl oder Tolyl oder -NX₅X₆ Pyrrolidinyl oder N-C₁-C₅-Alkyl-N-tetrahydrofurfurylamino bedeuten.

5. 2-Methylfluoranverbindung gemäss Anspruch 1, dadurch gekennzeichnet, dass sie der Formel 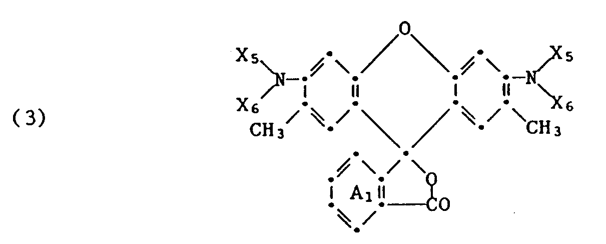 entspricht, worin A₁, X₅ und X₆ die im Anspruch 1 angegebene Bedeutung haben.

6. 2-Methylfluoranverbindung gemäss Anspruch 5, dadurch gekennzeichnet, dass in Formel (3) der Ring A₁ durch Halogen substituiert ist.

7. 2-Methylfluoranverbindung gemäss Anspruch 1, dadurch gekennzeichnet, dass sie der Formel 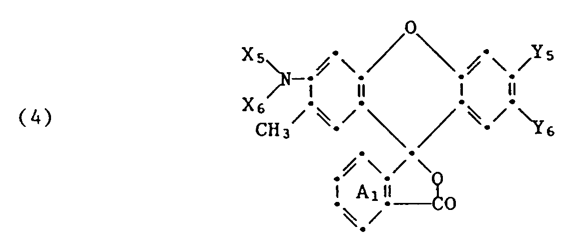 entspricht, worin A₁, X₅ und X₆ die in Anspruch 8 angegebene Bedeutung haben und Y₅ Wasserstoff, Halogen oder C₁-C₅-Alkyl und Y₆ Halogen oder C₁-C₅-Alkyl sind.

8. 2-Methylfluoranverbindung gemäss Anspruch 1, dadurch gekennzeichnet, dass sie der Formel 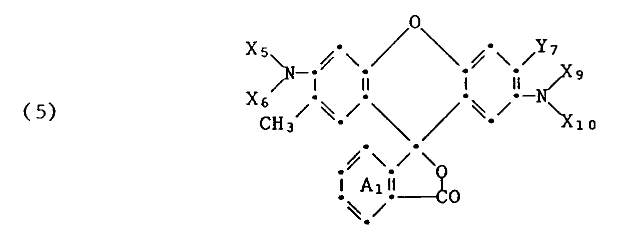 entspricht, worin A₁, X₅ und X₆ die in Anspruch 1 angegebene Bedeutung haben, Y₇ Wasserstoff, Halogen, C₁-C₅-Alkyl oder C₁-C₅-Alkoxy, X₉ C₁-C₈-Alkyl, Benzyl, Phenyl oder durch Halogen, Methyl, Methoxy, Carbomethoxy oder Trifluormethyl substituiertes Phenyl oder Benzyl und X₁₀ Wasserstoff, C₁-C₅-Alkyl, Benzyl oder durch Halogen, Methyl, Methoxy, Carbomethoxy oder Trifluormethyl substituiertes Benzyl bedeuten.

9. 2-Methylfluoranverbindung gemäss Anspruch 8, dadurch gekennzeichnet, dass in Formel (5) Y₇ Wasserstoff oder Methyl, X₉ Phenyl oder durch Halogen, Methyl oder Trifluormethyl substituiertes Phenyl und X₁₀ Wasserstoff bedeuten.

10. 2-Methylfluoranverbindung gemäss einem der Ansprüche 1 bis 9, dadurch gekennzeichnet, dass X₅ und X₆ je Methyl bedeuten.

11. Verfahren zur Herstellung von 3-basisch substituierten 2-Methylfluoranverbindungen gemäss Anspruch 1, dadurch gekennzeichnet, dass man
ein Mol eines Anhydrids der Formel 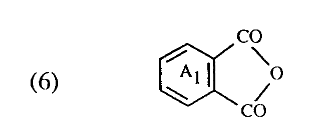 mit einen, Mol einer Verbindung der Formel 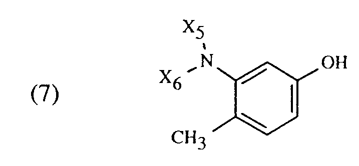 und einem Mol einer Verbindung der Formel 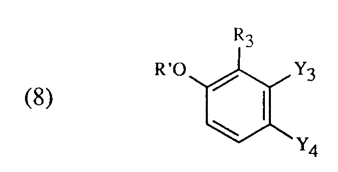 umsetzt, worin in Formeln (6), (7) und (8) A₁, X₅, X₆, Y₃, Y₄ und R₃ die im Anspruch 1 angegebenen Bedeutung haben, und R' Wasserstoff oder Methyl bedeutet.

12. Verfahren gemäss Anspruch 11, dadurch gekennzeichnet, dass man eine Ketosäureverbindung der Formel 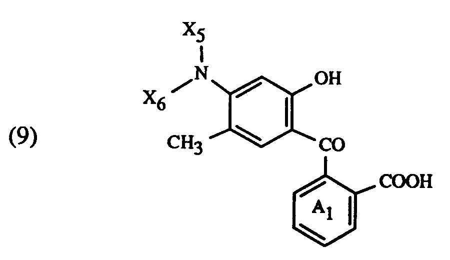 mit einer Verbindung der Formel (8) umsetzt, worin X₅, X₆ und A₁ die in Anspruch 11 gegebenen Bedeutung haben.

13. Verfahren gemäss Anspruch 11, dadurch gekennzeichnet, dass man die Verbindung der Formel (6) mit den Verbindungen der Formeln (7) und (8) gleichzeitig umsetzt.

14. Verwendung einer 3-basisch substituierten 2-Methylfluoranverbindung der in einem der Ansprüche 1 bis 10 angegebenen Formel als Farbbildner in einem druckempfindlichen oder wärmeempfindlichen Aufzeichnungsmaterial.

15. Druck- oder wärmeempfindliches Aufzeichnungsmaterial, dadurch gekennzeichnet, dass es in seinem Farbreaktantensystem als Farbbildner mindestens eine Fluoranverbindung der in einem der Ansprüche 1 bis 10 angegebenen Formel enthält.

16. Druckempfindliches Aufzeichnungsmaterial gemäss Anspruch 15, dadurch gekennzeichnet, dass es die Fluoranverbindung, gelöst in einem organischen Lösungsmittel, und mindestens einen festen Elektronenakzeptor enthält.

17. Druckempfindliches Aufzeichnungsmaterial gemäss einem der Ansprüche 15 und 16, dadurch gekennzeichnet, dass die Fluoranverbindung in Mikrokapseln eingekapselt ist.

18. Druckempfindliches Aufzeichnungsmaterial gemäss Anspruch 17, dadurch gekennzeichnet, dass die eingekapselte Fluoranverbindung in Form einer Schicht auf der Rückseite eines Uebertragungsblattes und der Elektronenakzeptor in Form einer Schicht auf der Vorderseite des Empfangsblattes vorhanden sind.

19. Wärmeempfindliches Aufzeichnungsmaterial gemäss Anspruch 15, dadurch gekennzeichnet, dass es in mindestens einer Schicht mindestens eine Fluoranverbindung der in einem der Ansprüche 1 bis 10 angegebenen Formel, einen Elektronenakzeptor und gegebenenfalls ein Bindemittel und/oder Wachs enthält.

20. Aufzeichnungsmaterial gemäss einem der Ansprüchen 15 bis 19, dadurch gekennzeichnet, dass die Fluoranverbindung gemeinsam mit einem oder mehreren anderen Farbbildnern enthalten ist.

## Claims

1. A 2-methylfluoran compound which has the formula 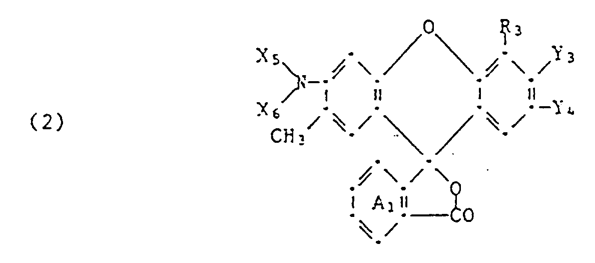 wherein
R₃ is hydrogen, halogen or C₁-C₅alkyl,
Y₃ is hydrogen, halogen, C₁-C₅alkyl, C₁-C₅alkoxy or 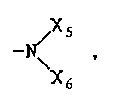 Y₄ is halogen, C₁-C₅alkyl or 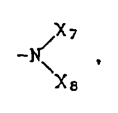 X₅, X₆, X₇ and X₈ are each independently of one another C₁-C₈alkyl, C₅-C₆cycloalkyl, tetrahydrofurfuryl, or benzyl or phenyl, each unsubstituted or substituted by halogen, cyano, C₁-C₅alkyl, trifluoromethyl, C₁-C₅alkoxy or C₁-C₅alkoxycarbonyl, and X₈ is also hydrogen, or the pairs of substituents (X₅ and X₆) and (X₇ and X₈), in each case together with the respective common nitrogen atom, are each independently of the other pyrrolidino, piperidino or morpholino, and the ring A₁ is unsubstituted or substituted by halogen, C₁-C₅alkoxycarbonyl or di-C₁-C₅alkylamino.

2. A 2-methylfluoran compound according to claim 1, wherein in formula (2) Y₃ is hydrogen, halogen or methyl and Y₄ is -NX₇X₈.

3. A 2-methylfluoran compound according to either of claims 1 and 2, wherein in formula (2) X₇ is C₁-C₈alkyl, benzyl, phenyl, or benzyl or phenyl each substituted by halogen, methyl, methoxy or trifluoromethyl, and X₈ is hydrogen, C₁-C₅alkyl, benzyl or benzyl which is substituted by halogen, methyl, methoxy or trifluoromethyl.

4. A 2-methylfluoran compound according to one of claims 1 to 3, wherein in formula (2) X₅ and X₆ are each independently of the other C₁-C₆alkyl, cyclohexyl, benzyl or tolyl, or -NX₅X₆ is pyrrolidinyl or N-C₁-C₅alkyl-N-tetrahydrofurfurylamino.

5. A 2-methylfluoran compound according to claim 1, which has the formula 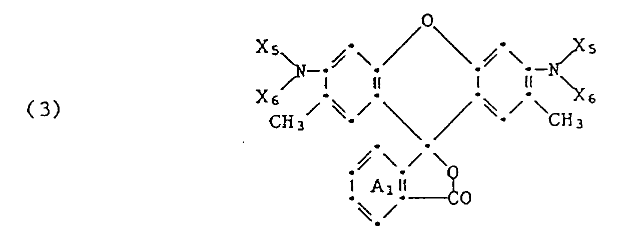 wherein A₁, X₅ and X₆ are as defined in claim 1.

6. A 2-methylfluoran compound according to claim 5, wherein in formula (3) the ring A₁ is substituted by halogen.

7. A 2-methylfluoran compound according to claim 1, which has the formula 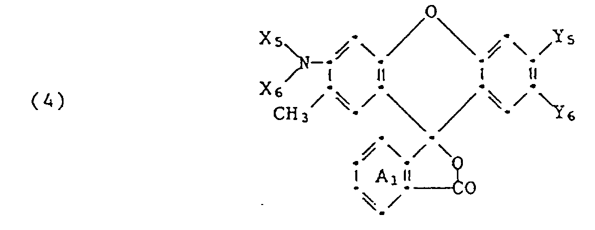 wherein A₁, X₅ and X₆ are as defined in claim 1 and Y₅ is hydrogen, halogen or C₁-C₅alkyl, and Y₆ is halogen or C₁-C₅alkyl.

8. A 2-methylfluoran compound according to claim 1, which has the formula 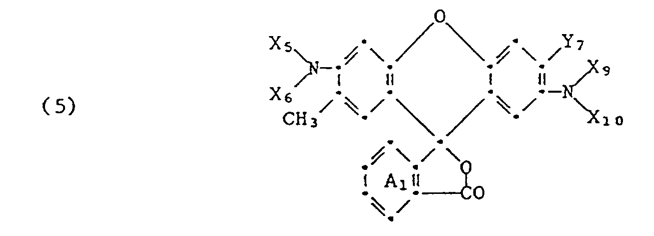 wherein A₁, X₅ and X₆ are as defined in claim 1, Y₇ is hydrogen, halogen, C₁-C₅alkyl or C₁-C₅alkoxy, X₉ is C₁-C₈alkyl, benzyl, phenyl, or phenyl or benzyl each substituted by halogen, methyl, methoxy, carbomethoxy or trifluoromethyl, and X₁₀ is hydrogen, C₁-C₅alkyl, benzyl or benzyl which is substituted by halogen, methyl, methoxy, carbomethoxy or trifluoromethyl.

9. A 2-methylfluoran compound according to claim 8, wherein in formula (5) Y₇ is hydrogen or methyl, X₉ is phenyl or phenyl which is substituted by halogen, methyl or trifluoromethyl, and X₁₀ is hydrogen.

10. A 2-methylfluoran compound according to one of claims 1 to 9, wherein X₅ and X₆ are each methyl.

11. A process for the preparation of a 2-methylfluoran compound which has a basic substituent in the 3 position, according to claim 1, which comprises reacting one mole of an anhydride of the formula 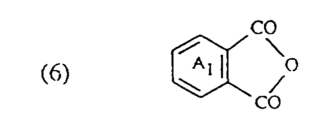 with one mole of a compound of the formula 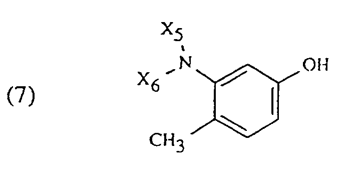 and one mole of a compound of the formula 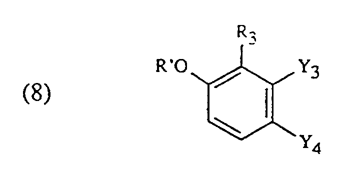 in which, in formulae (6), (7) and (8), A₁, X₅, X₆, Y₃, Y₄ and R₃ are as defined in claim 1 and R' is hydrogen or methyl.

12. A process according to claim 11, which comprises reacting a keto acid compound of the formula 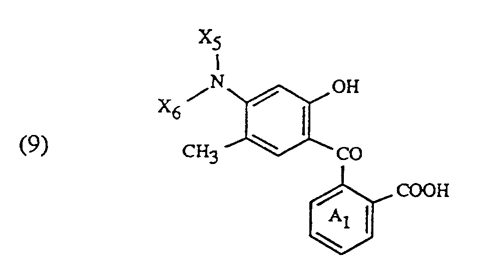 with a compound of the formula (8) in which X₅, X₆ and A₁ are as defined in claim 11.

13. A process according to claim 11, which comprises reacting a compound of the formula (6) simultaneously with compounds of the formulae (7) and (8).

14. The use of a 2-methylfluoran compound which has a basic substituent in the 3 position, of a formula given in one of claims 1 to 10, as a colour former in a pressure-sensitive or heat-sensitive recording material.

15. A pressure-sensitive or heat-sensitive recording material which comprises in its colour-reactant system, as colour former, at least one fluoran compound of a formula given in one of claims 1 to 10.

16. A pressure-sensitive recording material according to claim 15, which comprises the fluoran compound, dissolved in an organic solvent, and at least one solid electron acceptor.

17. A pressure-sensitive recording material according to either of claims 15 and 16, wherein the fluoran compound is encapsulated in microcapsules.

18. A pressure-sensitive recording material according to claim 17, wherein the encapsulated fluoran compound is present in the form of a layer on the back of a transfer sheet and the electron acceptor is present in the form of a layer on the face of the receiving sheet.

19. A heat-sensitive recording material according to claim 15, which comprises, in at least one layer, at least one fluoran compound of a formula given in one of claims 1 to 10, an electron acceptor and if desired a binder and/or wax.

20. A recording material according to one of claims 15 to 19, which comprises the fluoran compound together with one or more other colour formers.

## Revendications

1. Composé 2-méthylfluoranne, caractérisé en ce qu'il répond à la formule : 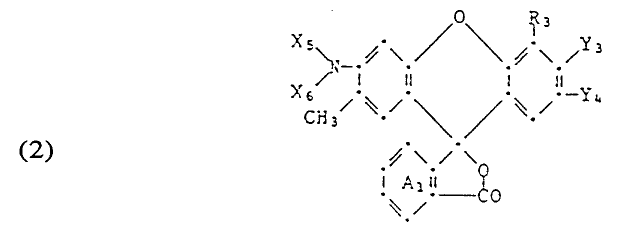 dans laquelle R₃ représente un atome d'hydrogène ou d'halogène, ou un groupe alkyle en C₁ à C₅,
Y₃ représente un atome d'hydrogène ou d'halogène, ou un groupe alkyle en C₁ à C₅, alcoxy en C₁ à C₅ ou 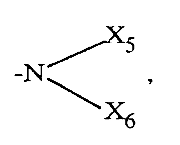 Y₄ représente un atome d'halogène ou un groupe alkyle en C₁ à C₅ ou 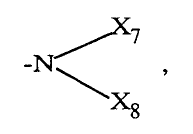 X₅, X₆, X₇ et X₈ représentent chacun, indépendamment l'un de l'autre, un groupe alkyle en C₁ à C₈, un groupe cycloalkyle en C₅ à C₆, un groupe tétrahydrofurfuryle ou un groupe benzyle ou phényle, non substitué ou substitué par un atome d'halogène ou un groupe cyano, alkyle en C₁ à C₅, trifluorométhyle, alcoxy en C₁ à C₅ ou alcoxy (C₁ à C₅)carbonyle, et X₈ peut également représenter un atome d'hydrogène, ou bien les paires de substituants (X₅ et X₆) et (X₇ et X₈) forment chacune, indépendamment l'une de l'autre, avec l'atome d'azote commun correspondant, un groupe pyrrolidino, pipéridino ou morpholino, et le noyau A₁ est non substitué ou substitué par un atome d'halogène ou un groupe alcoxy (C₁ à C₅)carbonyle ou dialkyl(C₁ à C₅)amino.

2. Composé 2-méthylfluoranne selon la revendication 1, caractérisé en ce que, dans la formule (2), Y₃ représente un atome d'hydrogène ou d'halogène, ou le groupe méthyle, et Y₄ représente un groupe -NX₇X₈.

3. Composé 2-méthylfluoranne selon la revendication 1 ou 2, caractérisé en ce que, dans la formule (2), X₇ représente un groupe alkyle en C₁ à C₈, un groupe benzyle, un groupe phényle, ou un groupe phényle ou benzyle, substitué par un atome d'halogène ou un groupe méthyle, méthoxy ou trifluorométhyle, et X₈ représente un atome d'hydrogène, un groupe alkyle en C₁ à C₅, un groupe benzyle ou un groupe benzyle substitué par un atome d'halogène ou un groupe méthyle, méthoxy ou trifluorométhyle.

4. Composé 2-méthylfluoranne selon l'une quelconque des revendications 1 à 3, caractérisé en ce que, dans la formule (2), X₅ et X₆ représentent chacun, indépendamment l'un de l'autre, un groupe alkyle en C₁ à C₆, cyclohexyle, benzyle ou tolyle, ou bien -NX₅X₆ représente un groupe pyrrolidinyle ou N-alkyl(C₁ à C₅)-N-tétrahydrofurfurylamino.

5. Composé 2-méthylfluoranne selon la revendication 1, caractérisé en ce qu'il répond à la formule : 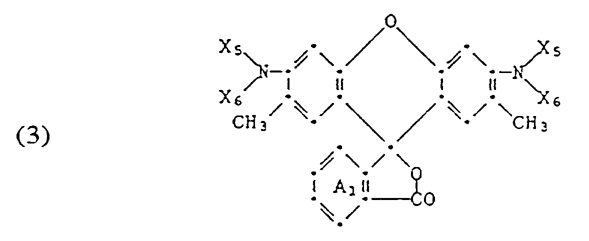 dans laquelle A₁, X₅ et X₆ ont les significations indiquées dans la revendication 1.

6. Composé 2-méthylfluoranne selon la revendication 5, caractérisé en ce que, dans la formule (3), le noyau A₁ est substitué par un atome d'halogène.

7. Composé 2-méthylfluoranne selon la revendication 1, caractérisé en ce qu'il répond à la formule : 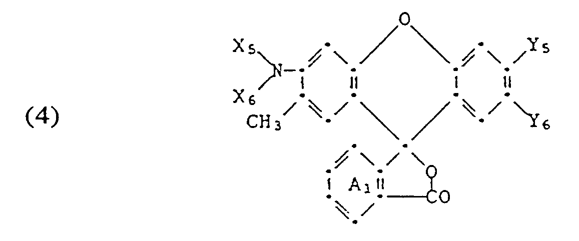 dans laquelle A₁, X₅ et X₆ ont les significations indiquées dans la revendication 1, et Y₅ représente un atome d'hydrogène ou d'halogène, ou un groupe alkyle en C₁ à C₅, et Y₆ représente un atome d'halogène ou un groupe alkyle en C₁ à C₅.

8. Composé 2-méthylfluoranne selon la revendication 1, caractérisé en ce qu'il répond à la formule : 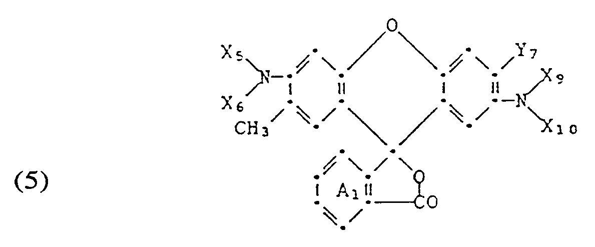 dans laquelle A₁, X₅ et X₆ ont les significations indiquées à la revendication 1, Y₇ représente un atome d'hydrogène ou d'halogène, ou un groupe alkyle en C₁ à C₅ ou alcoxy en C₁ à C₅, X₉ représente un groupe alkyle en C₁ à C₈, un groupe benzyle, un groupe phényle, ou un groupe phényle ou benzyle, substitué par un atome d'halogène ou un groupe méthyle, méthoxy, carbométhoxy ou trifluorométhyle, et X₁₀ représente un atome d'hydrogène ou un groupe alkyle en C₁ à C₅, un groupe benzyle ou un groupe benzyle substitué par un atome d'halogène ou un groupe méthyle, méthoxy, carbométhoxy ou trifluorométhyle.

9. Composé 2-méthylfluoranne selon la revendication 8, caractérisé en ce que, dans la formule (5), Y₇ représente un atome d'hydrogène ou un groupe méthyle, X₉ représente un groupe phényle ou un groupe phényle substitué par un atome d'halogène ou un groupe méthyle ou trifluorométhyle, et X₁₀ représente un atome d'hydrogène.

10. Composé 2-méthylfluoranne selon l'une quelconque des revendications 1 à 9, caractérisé en ce que X₅ et X₆ représentent chacun un groupe méthyle.

11. Procédé de préparation des composés 2-méthylfluoranne, substitués par un groupe basique en position 3, selon la revendication 1, caractérisé en ce que l'on fait réagir une mole d'un anhydride de formule : 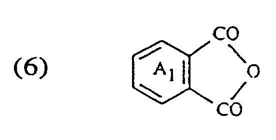 avec une mole d'un composé de formule : 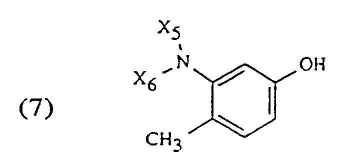 et une mole d'un composé de formule : 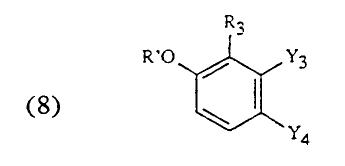 A₁,X₅,X₆,Y₃,Y₄ et R₃ ayant dans les formules (6), (7) et (8) les significations indiquées à la revendication 1, et R' représentant un atome d'hydrogène ou un groupe méthyle.

12. Procédé selon la revendication 11, caractérisé en ce que l'on fait réagir un cétoacide de formule : 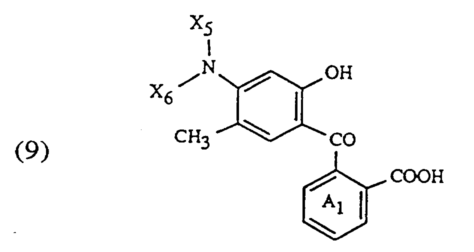 avec un composé de formule (8), X₅, X₆ et A₁ ayant les significations indiquées à la revendication 11.

13. Procédé selon la revendication 11, caractérisé en ce que l'on fait réagir le composé de formule (6) avec les composés de formules (7) et (8) simultanément.

14. Utilisation d'un composé 2-méthylfluoranne, substitué par un groupe basique en position 3, répondant à la formule indiquée dans l'une quelconque des revendications 1 à 10, en tant que formateur de colorant dans un matériau d'enregistrement, sensible à la pression ou sensible à la chaleur.

15. Matériau d'enregistrement sensible à la pression ou à la chaleur, caractérisé en ce qu'il contient dans son système de réactif pour la formation de couleur, en tant que formateur de colorant, au moins un composé fluoranne répondant à la formule indiquée dans l'une quelconque des revendications 1 à 10.

16. Matériau d'enregistrement sensible à la pression, selon la revendication 15, caractérisé en ce qu'il contient le composé fluoranne, dissous dans un solvant organique, et au moins un accepteur d'électrons solide.

17. Matériau d'enregistrement sensible à la pression, selon la revendication 15 ou 16, caractérisé en ce que le composé fluoranne est encapsulé dans des microcapsules.

18. Matériau d'enregistrement sensible à la pression, selon la revendication 17, caractérisé en ce que le composé fluoranne encapsulé est présent sous la forme d'une couche sur la face postérieure d'une feuille de transfert, et l'accepteur d'électrons est présent sous la forme d'une couche sur la face avant de la feuille réceptrice.

19. Matériau d'enregistrement sensible à la chaleur, selon la revendication 15, caractérisé en ce qu'il contient, dans au moins une couche, au moins un composé fluoranne répondant à la formule indiquée dans l'une quelconque des revendications 1 à 10, un accepteur d'électrons et éventuellement un liant et/ou une cire.

20. Matériau d'enregistrement selon l'une quelconque des revendications 15 à 19, caractérisé en ce qu'il contient le composé fluoranne et un ou plusieurs autres formateurs de colorant.
